# EUROPEAN PATENT APPLICATION

(11) **EP 4 837 522 A1**
(43) Date of publication of application: **11.11.2026**
(21) Application number: 26164914.9
(22) Date of filing: 16.03.2026
(51) Int. Cl.: A61B 17/128, A61B 17/00, A61B 17/122

(54) **DELIVERY DEVICE, CLIP DEVICE AND ASSEMBLY METHOD**

(30) Priority: 09.05.2025 CN 202510600338
(71) Applicant: Micro-Tech (Nanjing) Co., Ltd., Nanjing, Jiangsu 210032 (CN)
(72) Inventor: Tang, Zhi, Nanjing, Jiangsu 210032 (CN); Li, Chao, Nanjing, Jiangsu 210032 (CN)
(74) Representative: RavensPAT Patentanwälte Partnerschaft mbB

(57) **Abstract**

A delivery device, a clip device, and an assembly method are disclosed. The sheath assembly has a lateral hole, and a connecting assembly is disposed within the sheath assembly. When the transmission assembly moves along the channel toward the inside of the clip seat at the distal end, it can press the connecting assembly. Under the pressing of the transmission assembly, a portion of the connecting assembly can protrude from the lateral hole to the outside of the sheath assembly and connect with the clip seat. By controlling the movement of the transmission assembly, the delivery device and the clip assembly can be smoothly connected. Therefore, the delivery device and the clip assembly can be configured as separate structures, allowing for separate transportation and storage, reducing space occupation and transportation and storage costs. After releasing the clip assembly, the delivery device can be connected to another clip assembly using the same connection method to achieve repeated use and reduce operating costs.

## Description

The present disclosure claims the priority of the Chinese application No. 202510600338.9 filed on May 9, 2025, and the entire contents of which are incorporated herein by reference.

### FIELD OF THE INVENTION

The present disclosure belongs to the field of medical instruments, and in particular relates to a delivery device, a clip device, and an assembly method.

### BACKGROUND OF THE INVENTION

In the field of medical instruments, especially surgical instruments, endoscopic technology is being used more and more widely. An endoscope is a medical device with illumination and observation functions. By inserting it into the body, doctors can observe the internal conditions, allowing for diagnosis or treatment. In many surgeries, such as gastrointestinal surgeries and respiratory tract surgeries, hemostatic clips are needed to control bleeding. Hemostatic clips are common surgical instruments. By inserting the clip into the body and then controlling it to clamp blood vessels or tissues, hemostasis is achieved.

Currently, hemostatic clips are designed for single-use and consist of an integral structure. While this design is simple and convenient, it incurs high costs, which is not conducive to reducing surgical costs.

### SUMMARY OF THE INVENTION

### Objectives of the Invention

The present disclosure provides a delivery device to solve the technical problem of high cost of single-use hemostatic clips with integrated structure. Another objective of the present disclosure is to provide a clip device. A further objective of the present disclosure is to provide an assembly method.

### Technical Solution

A delivery device according to an embodiment of the present disclosure is provided, which is configured to deliver a clip assembly including a clip and a clip seat for accommodating at least a portion of the clip, wherein the delivery device includes:
a sheath assembly having a channel and a lateral hole, the channel extending through a proximal end and a distal end of the sheath assembly, and the lateral hole communicating with the channel;
a connecting assembly disposed within the channel and connected to the sheath assembly; and
a transmission assembly passing through the channel;
wherein the distal end of the sheath assembly is able to be inserted into the clip seat, and the transmission assembly is able to move along the channel toward the clip seat to connect with the clip; and
when the transmission assembly moves toward the inside of the clip seat, the transmission assembly is able to press the connecting assembly so that a portion of the connecting assembly protrudes from the lateral hole to the outside of the sheath assembly and connects with the clip seat.

In some embodiments, the transmission assembly is also capable of moving along the channel toward the proximal end of the sheath assembly to disengage from the clip.

When the transmission assembly moves toward the proximal end of the sheath assembly, the connecting assembly is able to disengage from the clip seat.

In some embodiments, the connecting assembly includes:
an elastic part having a fixed end and a movable end that are disposed opposite to each other, wherein the fixed end is fixed to the sheath assembly; and
a tongue part, which is connected to the movable end and extends from the movable end toward the lateral hole;
wherein when the transmission assembly moves toward the inside of the clip seat, the transmission assembly is capable of pressing the elastic part to cause it to elastically deform, thereby causing the tongue part to protrude from the lateral hole to the outside of the sheath assembly and connect with the clip seat.

In some embodiments, the sheath assembly has a central axis O.

The distance between the fixed end and the central axis O is greater than the distance between the movable end and the central axis O.

In some embodiments, the tongue part has a shoulder located within the sheath assembly.

The shoulder is able to contact a sidewall of the sheath assembly.

In some embodiments, the sheath assembly further has an assembly hole communicating with the channel.

The connecting assembly further includes a bending part connected to the fixed end, the bending part being disposed within the assembly hole and abutting against a hole wall of the assembly hole.

In some embodiments, the sheath assembly has a plurality of the lateral holes arranged circumferentially thereon.

The delivery device includes a plurality of the connecting assembly, and the tongue part of each connecting assembly is provided corresponding to one of the lateral holes.

In some embodiments, the sheath assembly further has an avoidance groove communicating with the channel, the avoidance grooves being located on opposite sides of the sheath assembly in its radial direction and extending along the extension direction of the sheath assembly; and the delivery device further includes:
an auxiliary assembly component, configured to move along the extension direction of the sheath assembly, wherein the auxiliary assembly component is provided with a clamping part disposed opposite to the avoidance groove, and the clamping part is configured to be able to enter the channel through the avoidance groove when pressed, so as to clamp the transmission assembly.

In some embodiments, the auxiliary assembly component includes a main body that is sleeved onto the sheath assembly and is movable along the extension direction of the sheath assembly, and the clamping part is connected to the main body.

The main body has a relief groove, which is provided corresponding to the avoidance groove.

At least a portion of the clamping part is disposed within the relief groove, and the clamping part is configured such that when pressed, at least a portion thereof is able to pass through the relief groove and enter the avoidance groove.

In some embodiments, the clamping part includes:
a drive arm having a connecting end and a free end disposed opposite to each other, the connecting end being connected to the main body; and from the connecting end to the free end, at least a portion of the drive arm extends in a direction gradually away from the transmission assembly; and
a pressing head, which is connected to the free end and extends from the free end toward the transmission assembly.

In some embodiments, the sheath assembly includes a sheath body and a transition cap arranged sequentially from its proximal end to its distal end and connected to each other, the transmission assembly passing through the sheath body and the transition cap, the lateral hole being formed on the transition cap, and at least a portion of the connecting assembly being located within the transition cap.

In some embodiments, an end of the transition cap away from the sheath body is the distal end of the sheath assembly, the transition cap is provided with a limiting step, and the lateral hole is located on the side of the limiting step closer to the distal end.

When the distal end of the sheath assembly is inserted into the clip seat, the lateral hole is located within the clip seat, and the limiting step is opposite to an edge of the clip seat.

In some embodiments, the transmission assembly includes a cable and a transmission head connected together, the cable being configured to drive the transmission head to move along the channel, and the transmission head being configured to press the connecting assembly and to connect with the clip.

In some embodiments, the transmission head includes:
a cone head configured to be inserted into the clip;
a base, which is connected to the cable and spaced apart from the cone head; and
a joint part connected between the base and the cone head;
wherein when the cone head is inserted into the clip, at least a portion of the clip is located between the cone head and the base and is in contact with the joint part.

Accordingly, a clip device as described in the embodiments of the present disclosure is provided, which includes:
the delivery device according to any of the above embodiments;
a clip assembly, wherein the clip assembly and the delivery device are configured as separate structures; and
a protective housing for assembling the clip assembly and the delivery device, wherein the protective housing includes a positioning assembly;
wherein the clip assembly is able to be received within the protective housing, and the delivery device is able to be inserted into the protective housing and positioned in conjunction with the positioning assembly to connect with the clip assembly.

In some embodiments, the clip assembly includes a clip and a clip seat that houses at least a portion of the clip, the clip being configured to connect to a transmission assembly of the delivery device, an inner peripheral surface of the clip seat being provided with a limiting groove, and a connecting assembly of the delivery device being able to be embedded in the limiting groove to connect to the clip seat.

In some embodiments, the clip includes a clip arm element and a latch connected together, the latch including at least two retaining units arranged about its central axis M, the retaining unit having a retaining part at an end away from the clip arm element.

The transmission head of the transmission assembly is able to be inserted between the at least two retaining units, and the retaining part is configured to limit the position of the transmission head so that the transmission head is connected to the latch.

In some embodiments, an internal space of the clip seat has a first section and a second section arranged sequentially along its axial direction, the first section being located on a side of the second section away from the clip arm element, and a radial dimension of the first section being greater than the radial dimension of the second section.

When the retaining part is located within the first section, the retaining parts of the at least two retaining units is able to be moved away from each other by a pressing force of the transmission head.

When the retaining part is located within the second section, the inner peripheral surface of the clip seat is able to contact the retaining unit to prevent the retaining parts of the at least two retaining units from moving away from each other.

In some embodiments, a receiving slot is formed between the at least two retaining units, and the transmission head is able to be inserted into the receiving slot. The retaining part is disposed at an opening of the receiving slot and protrudes radially inward.

In some embodiments, the latch further includes a hinge seat hinged to the clip arm element, and the retaining unit is disposed on the side of the hinge seat away from the clip arm element.

The retaining unit further includes a first retaining arm and a second retaining arm respectively connected to the hinge seat. The retaining part is disposed on the side of the first retaining arm and the second retaining arm away from the hinge seat and connects the first retaining arm and the second retaining arm.

In some embodiments, when the transmission head is inserted between the at least two retaining units, the retaining part is located between the cone head and the base of the transmission head and is in contact with the joint part.

In some embodiments, the transmission head is capable of driving the clip assembly to rotate relative to the connecting assembly.

In some embodiments, the latch is configured as an elastic structure.

In some embodiments, the clip arm element includes a first clip arm and a second clip arm hinged to the latch, both the first clip arm and the second clip arm being provided with a sliding groove. A pin is provided in the clip seat, and passes through the sliding groove.

The sliding groove is provided with an open position A, a closed position B, and a locking position C. The transmission assembly drives the clip arm element to move through the latch, so that the pin is able to reciprocate between the open position A and the closed position B. In the open position A, the first clip arm and the second clip arm are open, and in the closed position B, the first clip arm and the second clip arm are closed.

The locking position C is located on the side of the closed position B away from the open position A. The first clip arm is provided with a first protrusion in its sliding groove, and the second clip arm is provided with a second protrusion in its sliding groove opposite to the first protrusion. The first protrusion and the second protrusion are located between the locking position C and the closed position B. The transmission assembly drives the clip arm element to move toward the proximal end through the latch, so that the pin presses the first protrusion and the second protrusion and moves to the locking position C, where the first clip arm and the second clip arm are locked.

In some embodiments, the first clip arm is provided with a first avoidance notch, and the second clip arm is provided with a second avoidance notch. The pin presses the first protrusion and the second protrusion, which is able to cause the first protrusion to deform toward the first avoidance notch and the second protrusion to deform toward the second avoidance notch.

In some embodiments, the protective housing further includes:
a housing assembly, internally defining a receiving slot and an assembly slot, wherein the receiving slot is configured to accommodate the clip assembly, and the assembly slot is configured to allow the delivery device to be inserted into the receiving slot therethrough;
wherein the positioning assembly is connected to the housing assembly and includes a first positioning part and a second positioning part arranged opposite to each other, wherein the first positioning part and the second positioning part are disposed between the receiving slot and the assembly slot, and are configured to engage with the sheath assembly in the delivery device to position the sheath assembly when the delivery device is inserted into the receiving slot.

Accordingly, embodiments of the present disclosure also provide an assembly method applied to the clip device described in any of the above embodiments, wherein the assembly method includes:
inserting the delivery device into the protective housing so that the positioning assembly of the protective housing cooperates with the delivery device to position the delivery device;
manipulating a handle mechanism of the delivery device to insert the transmission assembly of the delivery device into the clip seat of the clip assembly and connect it with the clip, and the clip assembly is received in the protective housing in a closed state; and
completing the assembly by pulling the delivery device out of the protective housing and taking the clip assembly out together.

### Beneficial Effects

The delivery device provided in the embodiment of the present disclosure is configured to deliver a clip assembly including a clip and a clip seat for accommodating at least a portion of the clip. The delivery device includes: a sheath assembly having a channel and a lateral hole, the channel extending through the proximal end and the distal end of the sheath assembly, and the lateral hole communicating with the channel; a connecting assembly disposed within the channel and connected to the sheath assembly; and a transmission assembly passing through the channel. The distal end of the sheath assembly is able to be inserted into the clip seat, and the transmission assembly is able to move along the channel toward the clip seat to connect with the clip. When the transmission assembly moves toward the inside of the clip seat, the transmission assembly is able to press the connecting assembly so that a portion of the connecting assembly protrudes from the lateral hole to the outside of the sheath assembly and connect with the clip seat. In embodiment of the present disclosure, the sheath assembly has the lateral hole, and the connecting assembly is connected within the sheath assembly. When the transmission assembly moves along the channel toward the inside of the distal clip seat, it can press the connecting assembly. The connecting assembly is pressed by the transmission assembly, and a portion of it can protrude from the lateral hole to the outside of the sheath assembly and connect with the clip seat. In other words, by controlling the movement of the drive assemblies, the delivery device and the clip assembly can be smoothly connected. Therefore, the delivery device and the clip assembly can be designed as separate structures, allowing them to be transported and stored separately, reducing space occupation and transportation and storage costs. After releasing the clip assembly, the delivery device can be connected to another clip assembly using the same connection method to achieve repeated use and reduce operating costs.

The clip device according to the embodiment of the present disclosure may have all the technical features and effects of the above-mentioned delivery device, which will not be repeated here.

### BRIEF DESCRIPTION OF THE DRAWINGS

To more clearly illustrate the technical solutions in the embodiments of the present disclosure, the accompanying drawings used in the description of the embodiments will be briefly introduced below. Obviously, the accompanying drawings described below are only some embodiments of the present disclosure. For those skilled in the art, other drawings can be obtained based on these drawings without creative effort.
FIG 1 is a structural diagram of the clip assembly and the delivery device in the clip device provided in an embodiment of the present disclosure.
FIG 2 is a structural diagram of the clip assembly in an embodiment of the present disclosure.
FIG 3 is a sectional view of the structure of the clip assembly along line E-E in FIG 2.
FIG 4 is a diagram of partial structure of the delivery device provided in an embodiment of the present disclosure.
FIG 5 is a partial enlarged diagram of the structure of region A in FIG 4.
FIG 6 is a sectional view of the structure along line A-A in FIG 5.
FIG 7 is a sectional view of partial structure of the connecting assembly of the delivery device provided in an embodiment of the present disclosure in an extended state.
FIG 8 is a diagram of partial structure of the delivery device and the clip seat of the clip assembly in a connecting state provided in an embodiment of the present disclosure.
FIG 9 is a sectional view of the structure in FIG 8 along line C-C when the retaining part is located in the first section.
FIG 10 is a sectional view of the structure in FIG 8 along C-C line when the retaining part is located in the second section.
FIG 11 is a diagram of partial structure of the clip device provided in an embodiment of the present disclosure when the clip assembly is in an open state.
FIG 12 is a sectional view of the structure in FIG 11 along line B-B.
FIG 13 is a structural diagram of the connecting assembly in the delivery device provided in an embodiment of the present disclosure.
FIG 14 is a schematic diagram of the connecting assembly in the delivery device provided in another embodiment of the present disclosure.
FIG 15 is a diagram of partial structure of the connecting assembly of the delivery device provided in an embodiment of the present disclosure in an extended state.
FIG 16 is a side view of the structure in FIG 15.
FIG 17 is a sectional view of the structure in FIG 16 along line D-D.
FIG 18 is a diagram of partial structure of the delivery device provided in an embodiment of the present disclosure.
FIG 19 is a side view of partial structure at the auxiliary assembly component in the embodiment of FIG 18.
FIG 20 is a diagram of partial structure of the sheath assembly in the embodiment of FIG 19 after removing the auxiliary assembly component.
FIG 21 is a sectional view of partial structure showing the connecting state of the transmission assembly of the delivery device and the clip of the clip assembly according to an embodiment of the present disclosure.
FIG 22 is a structural diagram of the transmission head in an embodiment of the present disclosure.
FIG 23 is a structural diagram of the clip assembly in an embodiment of the present disclosure.
FIG 24 is a diagram of the connection structure between the transmission head and the latch in an embodiment of the present disclosure.
FIG 25 is a structural diagram of the connection structure between the transmission head and the latch in FIG 24 from another perspective.
FIG 26 is a side view of the connection structure between the transmission head and the latch in FIG 24.
FIG 27 is a sectional view of a structure along line F-F in FIG 26.
FIG 28 is a sectional view of another structure along line F-F in FIG 26.
FIG 29 is a sectional view of a third structure along line F-F in FIG 26.
FIG 30 is a sectional view of a fourth structure along line F-F in FIG 26.
FIG 31 is a sectional view of a fifth structure along line F-F in FIG 26.
FIG 32 is a structural diagram of the latch according to another embodiment of the present disclosure.
FIG 33 is a sectional view of partial structure showing the connecting state of the transmission assembly of the delivery device and the clip of the clip assembly according to an embodiment of the present disclosure.
FIG 34 is a structural diagram of the protective housing of the clip device in an embodiment of the present disclosure.
FIG 35 is an exploded structural view of the parts of the clip device during the assembly process in an embodiment of the present disclosure.

List of reference numerals in the attached drawings:
100 - Delivery Device;
110 - Sheath Assembly;
111 - Sheath Body;
112 - Transition Cap;
1121 - Side Wall;
1122 - Limiting Step;
1123 - Limiting Recess;
113 - Channel;
114 - Lateral Hole;
115 - Proximal End;
116 - Distal End;
117 - Assembly Hole;
1171 - Hole Wall;
118 - Avoidance Groove;
120 - Connecting Assembly;
121 - Elastic Part;
1211 - Fixed End;
1212 - Movable End;
122 - Tongue Part;
123 - Bending Part;
1221 - Shoulder;
130 - Transmission Assembly;
131 - Cable;
132 - Transmission Head;
1321 - Cone Head;
1322 - Joint Part;
1323 - Base;
140 - Auxiliary Assembly Component;
141 - Clamping Part;
1411 - Drive Arm;
1412 - Pressing Head;
1413 - Connecting End;
1414 - Free End;
142 - Main Body;
1421 - Relief Groove;
150 - Handle Mechanism;
200 - Clip Assembly;
210 - Clip;
211 - Clip Arm Element;
211a - First Clip Arm;
211b - Second Clip Arm;
212 - Latch;
2121 - Outer Peripheral Surface;
2122 - Accommodation Slot;
2123 - Opening;
213 - Retaining Unit;
2131 - Retaining Part;
2132 - First Retaining Arm;
2133 - Second Retaining Arm;
217 - Hinge Seat;
214 - Sliding Groove;
215a - First Protrusion;
215b - Second Protrusion;
216a - First Avoidance Notch;
216b - Second Avoidance Notch;
220 - Clip Seat;
221 - Inner Peripheral Surface;
222 - Limiting Groove;
223 - First Section;
224 - Second Section;
225 - Pin;
300 - Protective Housing;
310 - Housing Assembly;
311 - Receiving Slot;
312 - Assembly Slot;
3121 - Guide Surface;
320 - Positioning Assembly;
321 - First Positioning Part;
322 - Second Positioning Part.

### DETAILED DESCRIPTION OF THE EMBODIMENTS

The technical solutions of the embodiments of the present disclosure will be clearly and completely described below with reference to the accompanying drawings. Obviously, the described embodiments are only a part of the embodiments of the present disclosure, and not all of them. All other embodiments obtained by those skilled in the art based on the embodiments of the present disclosure without creative effort are within the scope of protection of the present disclosure.

In the description of the present disclosure, it should be understood that the terms "proximal end" and "distal end" are relative to the operator (doctor). "Proximal end" refers to the end closer to the operator, and "distal end" refers to the end further away from the operator relative to the "proximal end", i.e., closer to the patient. The terms "upper," "lower," "inner," and "outer," etc., indicate the orientation or positional relationship based on the orientation or positional relationship shown in the accompanying drawings, and are only for the convenience of describing the present disclosure and simplifying the description, and do not indicate or imply that the device or apparatus referred to must have a specific orientation, or be constructed and operated in a specific orientation, and therefore should not be construed as a limitation of the present disclosure. "A plurality" means two or more, and "at least one" can mean one, two, or more, unless otherwise explicitly specified. The terms "mounted," "connected," and "coupled" should be interpreted broadly, for example, they can refer to a fixed connection, a detachable connection, or an integral connection; they can refer to a direct connection or an indirect connection through an intermediate medium; they can refer to the internal communication of two devices or the interaction between two devices, unless otherwise explicitly specified. The terms "first" and "second" are used for descriptive purposes only and should not be construed as indicating or implying relative importance or implicitly specifying the number of technical features indicated. Therefore, a feature defined as "first" or "second" may explicitly or implicitly include one or more of that feature.

As an introduction to the present disclosure, a hemostatic clip is first described, which consists of a clip assembly and a delivery device. These two parts are an integral unit for disposable use. While this design is simple and convenient, in practical use, since both the clip assembly and the delivery device are disposable, the cost is high, which is not conducive to large-scale use of the product. Moreover, since the clip assembly and the delivery device are integrated, if the clip assembly is damaged during use, the entire hemostatic clip needs to be replaced, which undoubtedly increases medical costs. Furthermore, since the clip assembly and the delivery device are integrated, they occupy a large space during transportation and storage, which also increases transportation and storage costs.

In addition, regarding assembled hemostatic clips, current solutions also have many problems. For example, in patent CN100475165C, this solution has the following problems:
1) In this technology, during the assembly process of the clip assembly, the protective housing needs to be pressed by hand to fix the delivery device and the clip assembly, and then the connection head is pushed to complete the assembly. This method is relatively complex to operation, especially in the tense surgical environment, which makes it more difficult to operate and increases the difficulty of assembly.
2) The clip arm element in this solution cannot be repeatedly opened and closed, because there are no elastic piece to connect the delivery device and the clip assembly in this solution, which greatly increases the difficulty and inconvenience of the operation in actual use.
3) The clip arm element in this solution cannot be rotated, which will greatly increase the difficulty of the operation and reduce the efficiency of the operation in actual use.
4) When changing to the second clip assembly, since the "corpse" of the previous clip assembly remains at the front end of the connection head, it needs to be manually removed. Only after removal can the next clip assembly be assembled, which also increases the difficulty of assembly and reduces surgical efficiency.

In addition, as in patent CN103989500B, this solution has the following problems:
1) The clip assembly cannot be reloaded, which contradicts the concept of sustainable development in the current context of advocating green environmental protection. In particular, the EU's PPWR regulation requires reducing packaging waste and promoting the recycling and reuse of packaging materials. Therefore, if the solution described in CN103989500B is implemented, the remaining delivery device will be discarded after the clip assembly is used, which not only conform to sustainable development but also increases the burden on patients.

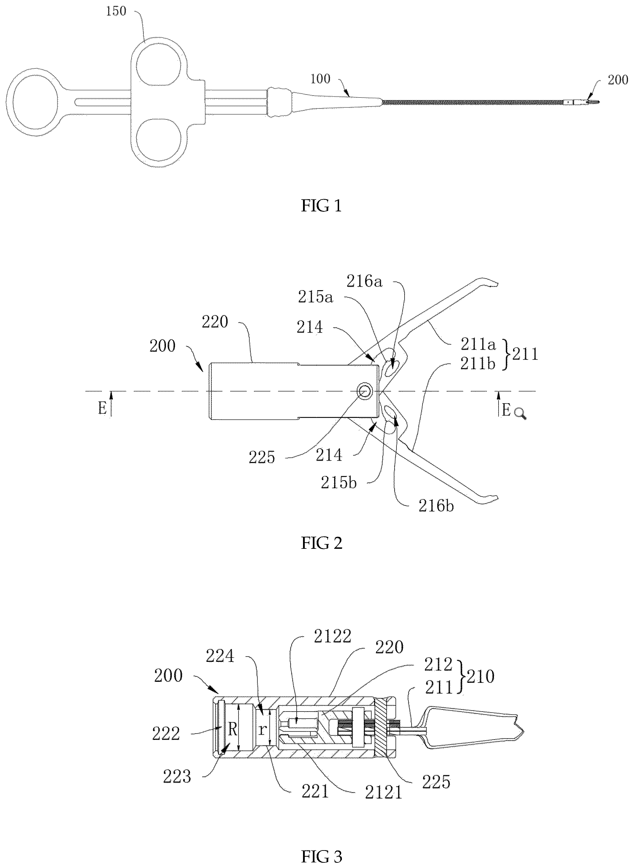

In view of this, the present disclosure provides a clip device that can be used in endoscopic surgery for clamping and closing tissues and wounds. As shown in FIG 1, the clip device mainly includes a delivery device 100 and a clip assembly 200. The delivery device 100 and the clip assembly 200 are separate structures, and the delivery device 100 is configured to be capable of connecting with the clip assembly 200 to deliver the clip assembly 200. Specifically, by setting the clip assembly 200 and the delivery device 100 as two independent parts, where the clip assembly 200 is for single use and the delivery device 100 is for repeated use, product costs can be greatly reduced. Furthermore, it also solves the problem that the entire clip device needs to be replaced when the clip assembly 200 is damaged. By setting the clip assembly 200 and the delivery device 100 as two independent parts, only the clip assembly 200 needs to be replaced when it is damaged, instead of replacing the entire clip device, allowing the delivery device 100 to continue to be used, thereby reducing medical costs. Furthermore, it also solves the problem of large space occupation during transportation and storage caused by the integration of the clip assembly 200 and the delivery device 100. By setting the clip assembly 200 and the delivery device 100 as two independent parts, they can be transported and stored separately, thereby reducing transportation and storage costs.

Specifically, FIG 1 shows a state where the delivery device 100 and the clip assembly 200 are connected together. The delivery device 100 and clip assembly 200 can also be in a separated state for separated storage and transport. When needed, the delivery device 100 and clip assembly 200 are connected. For example, the clip assembly 200 is housed and secured within a protective housing, and the delivery device 100 is inserted into the protective housing and connected to the clip assembly 200. Once connected, the delivery device 100 can be used to deliver the clip assembly 200 through an endoscopic channel into a patient's body to clamp the wound/tissue requiring treatment.

Referring to FIGS 2 and 3 first, the clip assembly 200 includes a clip 210 and a clip seat 220. At least a portion of the clip 210 is accommodated within the clip seat 220. The clip 210 includes a clip arm element 211 for clamping tissue/wound and a latch 212 connected to the clip arm element 211.

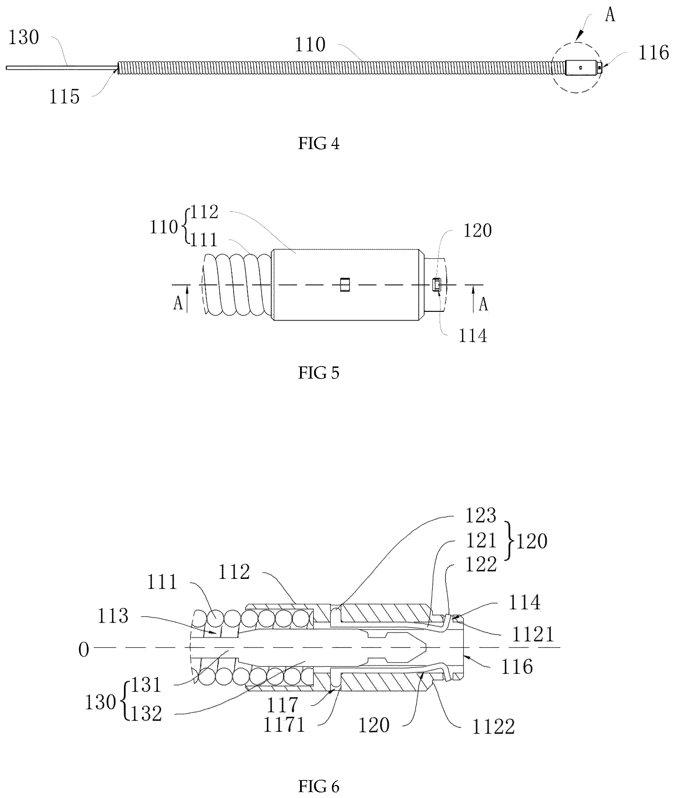

Referring to FIGS 4, 5, and 6 together, the embodiment of the present disclosure also provides a delivery device 100 for delivering the clip assembly 200. In an embodiment of the present disclosure, the delivery device 100 mainly includes a sheath assembly 110, a connecting assembly 120, and a transmission assembly 130.

One end of the sheath assembly 110 is its proximal end 115, and the other end is its distal end 116. The sheath assembly 110 is used to deliver the assembled clip assembly 200, located at its distal end 116, through the endoscopic channel into the patient's body. The sheath assembly 110 has a channel 113 and a lateral hole 114. The channel 113 is a cavity formed within the sheath assembly 110, penetrating both the proximal end 115 and the distal end 116, giving the sheath assembly 110 a generally tubular structure. Thus, the channel 113 of the sheath assembly 110 can be used to pass through the transmission assembly 130, and the sheath assembly 110 can guide and protect the transmission assembly 130 passing through its channel 113. The lateral hole 114 is formed on the side wall 1121 of the sheath assembly 110 and can extend generally radially along the sheath assembly 110, or it can be extended at a required inclination angle. The lateral hole 114 communicates with the channel 113 and penetrates the side wall 1121 of the sheath assembly 110.

The connecting assembly 120 is disposed within the channel 113 and is connected to the sheath assembly 110, meaning that the connecting assembly 120 is mounted on the sheath assembly 110 and can move with the sheath assembly within the endoscope channel. The connecting assembly 120 is used to engage with the clip seat 220 of the clip assembly 200 to connect the clip assembly 200 to the distal end 116 of the sheath assembly 110. Specifically, a portion of the connecting assembly 120 (such as the tongue part 122) can protrude through the lateral hole 114 and outside the sheath assembly 110 to engage with the clip seat 220.

Referring to FIGS 6, 7, 9, and 10 together, the distal end 116 of the sheath assembly 110 can be inserted into the clip seat 220, and the transmission assembly 130 can move along the channel 113 toward the clip seat 220 so that one end of the transmission assembly 130 is connected to the clip 210 of the clip assembly 200. As the transmission assembly 130 moves toward the clip seat 220, it can press the connecting assembly 120 so that a portion of the connecting assembly 120 protrudes from the lateral hole 114 out of the sheath assembly 110 and connects to the clip seat 220.

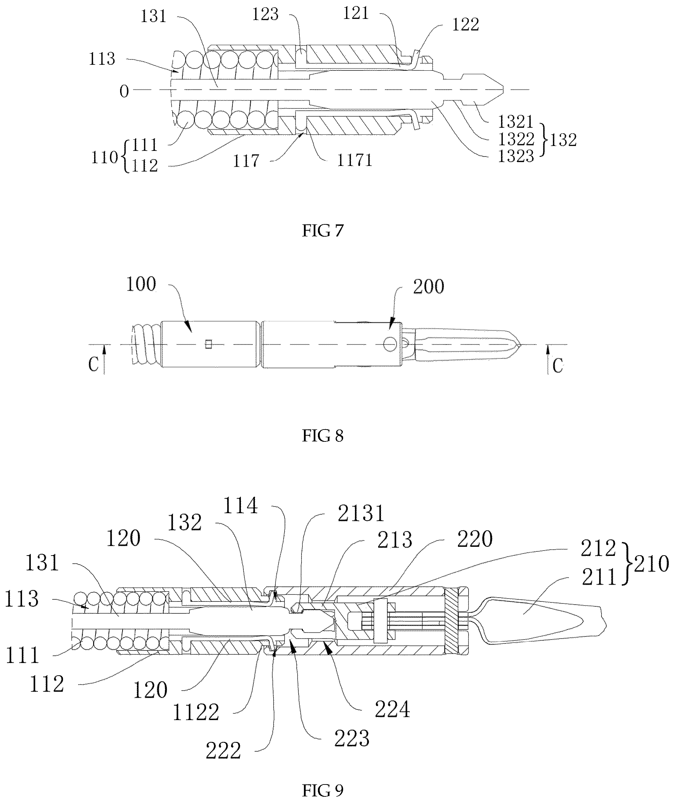

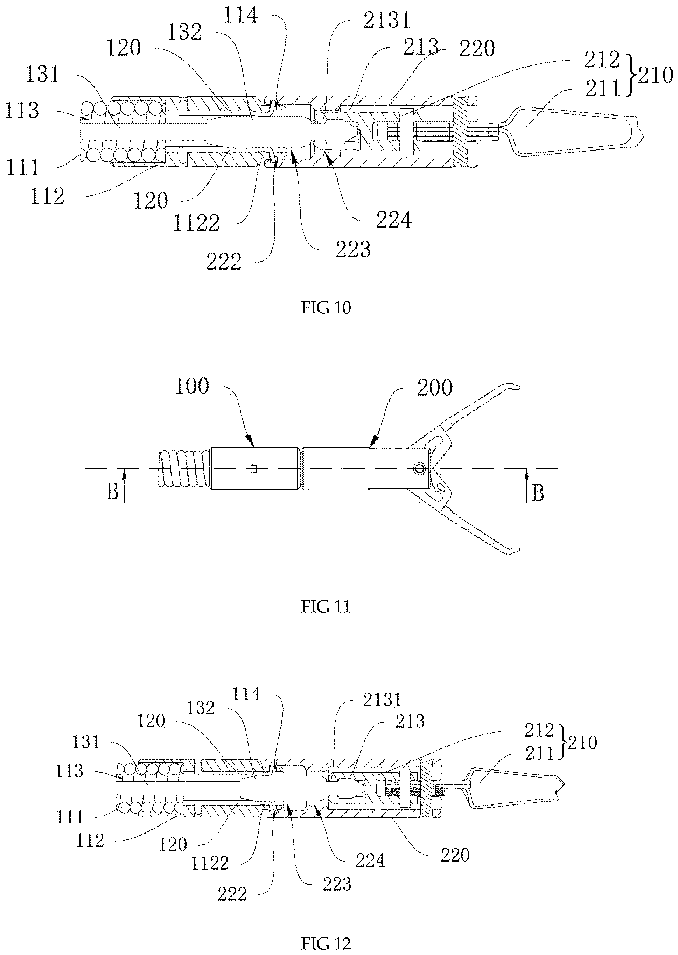

Specifically, referring to FIGS 6 and 7 together, by driving the transmission assembly 130, the transmission assembly 130 can move along the channel 113 toward the side where the proximal end 115 of the sheath assembly 110 is located or toward the side where the distal end 116 is located. As the transmission assembly 130 moves toward the side where the distal end 116 is located, its front end gradually moves outside the channel 113, pressing the connecting assembly 120 located within the channel 113 in the process. As shown in FIG 7, the connecting assembly 120 is subjected to radial pressing force, and its tongue part 122 can move radially outward and pass out through the lateral hole 114, thus protruding outside the sheath assembly 110. Referring to FIGS 8 and 9 together, when assembling the delivery device 100 with the clip assembly 200, the distal end 116 of the sheath assembly 110 can be inserted into the clip seat 220. The transmission assembly 130 moves toward the side where the distal end 116 is located, pressing the connecting assembly 120 so that its tongue part 122 protrudes from the lateral hole 114 to the outside of the sheath assembly 110 and engages with the clip seat 220. Optionally, as the distal end 116 of the sheath assembly 110 is inserted into the clip seat 220, the lateral hole 114 also enters the inner cavity of the clip seat 220. The tongue part 122 can be embedded into the limiting groove 222 provided on the inner peripheral surface 221 of the clip seat 220 to achieve engagement with the clip seat 220; or it can be engaged with the limiting boss provided on the clip seat 220; or it can be inserted into the limiting hole provided on the clip seat 220 to achieve engagement with the clip seat 220. Referring to FIGS 9 and 10 together, as the transmission assembly 130 moves toward the side where the distal end 116 is located, its front end gradually moves into the clip seat 220, enabling it to connect with the clip 210 of the clip assembly 200. Furthermore, it can drive the clip 210 to open and close, completing the operations required for surgery.

It is understood that in the embodiment of the present disclosure, when the transmission assembly 130 moves along the channel 113 into the inside of the clip seat 220 at the distal end 116, it presses the connecting assembly 120. The connecting assembly 120, under the pressing of the transmission assembly 130, allows a portion of its structure to protrude from the lateral hole 114 to the outside of the sheath assembly 110 and connect with the clip seat 220. In other words, when connecting the delivery device 100 to the clip assembly 200, by controlling the movement of the transmission assembly 130 toward the distal end 116, the delivery device 100 and the clip assembly 200 can be smoothly connected. Therefore, the delivery device 100 and the clip assembly 200 can be configured as separate structures, and can be connected together when needed. The delivery device 100 and the clip assembly 200 can be transported and stored separately, reducing space occupation and transportation and storage costs. After releasing the clip assembly 200, the delivery device 100 can be connected to another clip assembly 200 through the same connection method to achieve repeated use of the delivery device 100 and reduce usage costs.

Referring to FIGS 12, 10, and 9 in sequence, in some embodiments, the transmission assembly 130 is further capable of moving along the channel 113 toward the proximal end 115 of the sheath assembly 110 to separate from the clip 210. Specifically, referring to FIGS 12, 11, 10, 9, and 8, as the transmission assembly 130 moves along the channel 113 toward the proximal end 115 of the sheath assembly 110, it can drive the clip 210 from an open state to a closed state, achieving clamping of the tissue/wound. Then, as the transmission assembly 130 continues to move toward the proximal end 115, it gradually separates from the clip 210 and gradually disengages from the inner cavity of the clip seat 220, retracting from the distal end 116 back into the channel 113. In the case where the transmission assembly 130 moves toward the proximal end 115 of the sheath assembly 110, the pressure exerted on the connecting assembly 120 gradually decreases, causing the connecting assembly 120 to gradually retract into the sheath assembly 110 to separate from the clip seat 220, completing the release of the clip assembly 200. Specifically, referring to FIGS 7 and 6 in sequence, as the transmission assembly 130 moves toward the proximal end 115, the pressure exerted on the connecting assembly 120 gradually decreases, allowing the tongue part 122 of the connecting assembly 120 to move through the lateral hole 114 toward the side where the channel 113 is located, that is, gradually approaching the central axis O, gradually retracting into the lateral hole 114 or even into the channel 113, thereby achieving separation from the clip seat 220. This allows the connected clip assembly 200 to be released, making the delivery device 100 reusable to connect with another clip assembly 200 for delivery.

As shown in FIG 1, in some embodiments, the delivery device 100 further includes a handle mechanism 150, which is connected to the proximal end 115 of the sheath assembly 110 and to the transmission assembly 130. By manipulating the handle mechanism 150, the transmission assembly 130 can be driven to move toward the side where the proximal end 115 is located or toward the side where the distal end 116 is located. Thus, by manipulating the handle mechanism 150, the connection between the delivery device 100 and the clip assembly 200 can be achieved, and the delivery of the clip assembly 200 through the endoscope channel into the patient's body can be achieved, further achieving actions such as opening, closing, locking, and releasing of the clip assembly 200.

Referring again to FIGS 4, 5, and 6, in some embodiments, the sheath assembly 110 includes a sheath body 111 and a transition cap 112 arranged sequentially and connected to each other from its proximal end 115 to its distal end 116, wherein the sheath body 111 can be a coil tube, i.e., a pipe structure formed by spirally winding metal wire. One end thereof is connected to the handle mechanism 150, and the end connected to the handle mechanism 150 corresponds to the proximal end 115, and the other end is connected to the transition cap 112. The transition cap 112 can be made of materials such as metal or medical plastic, and its end away from the sheath body 111 corresponds to the distal end 116. The channel 113 extends from the proximal end 115 to the distal end 116, penetrating the sheath body 111 and the transition cap 112. That is, the channel 113 has at least two parts: one part is the internal space of the sheath body 111, and the other part is the internal space of the transition cap 112, both communicating with each other to form the channel 113. The transmission assembly 130 passes through the sheath body 111 and the transition cap 112. In this embodiment, the lateral hole 114 is formed on the transition cap 112, and at least a portion of the connecting assembly 120 is located within the transition cap 112. By forming the lateral hole 114 on the transition cap 112, making it closer to the distal end 116, so that the connecting assembly 120, which extends out from the lateral hole 114, can more easily connect with the clip seat 220 when the distal end 116 is inserted into the clip seat 220. Furthermore, the transition cap 112 is generally more rigid than the sheath body 111, and forming the lateral hole 114 on the transition cap 112 is easier than on the sheath body 111, which can significantly reduce the difficulty of processing.

Optionally, referring to FIGS 6 and 9 together, the end of the transition cap 112 away from the sheath body 111 is the distal end 116 of the sheath assembly 110. The transition cap 112 is provided with a limiting step 1122, and the lateral hole 114 is located on the side of the limiting step 1122 near the distal end 116. When the distal end 116 of the sheath assembly 110 is inserted into the clip seat 220, the lateral hole 114 is located within the clip seat 220, and the limiting step 1122 is opposite to the edge of the clip seat 220. The edge of the clip seat 220 is the end face of the clip seat 220 near the transition cap 112 in FIG 9. By providing the limiting step 1122, the distance that the distal end 116 of the transition cap 112 is inserted into the cavity of the clip seat 220 can be limited by the cooperation between the limiting step 1122 and the edge of the clip seat 220, thereby aligning and positioning the lateral hole 114 with the matching assembly structure provided in the clip seat 220. For example, FIG 9 shows that a limiting groove 222 is provided on the inner side of the clip seat 220. Through the cooperative limiting by the limiting step 1122 and the edge of the clip seat 220, the position of the lateral hole 114 entering the inner cavity of the clip seat 220 corresponds to the limiting groove 222, improving the accuracy of the assembly of the delivery device 100 and the clip assembly 200.

Referring to FIG 6, in some embodiments, the transmission assembly 130 includes a cable 131 and a transmission head 132 connected together. The cable 131 is configured to drive the transmission head 132 to move along the channel 113. The transmission head 132 is configured to press the connecting assembly 120 and connect to the clip 210. One end of the cable 131 can be connected to a handle mechanism 150, which drives the cable 131 to move. The transmission head 132 is connected to the other end of the cable 131, and provided thereon a structure for connecting to the clip 210 and a structure for pressing the connecting assembly 120. Optionally, the peripheral wall of the transmission head 132 is provided with a protruding portion (with a larger radial dimension) protruding toward the side wall 1121 of the sheath assembly 110. This protruding portion is used to press the connecting assembly 120. When the cable 131 moves toward the distal end 116, it pushes the transmission head 132 to move toward the distal end 116 together. The protruding portion on its peripheral wall contacts the connecting assembly 120 and presses the connecting assembly 120 as it moves, causing part of its structure to protrude outward from the lateral hole 114. Optionally, the front end of the transmission head 132 is provided with a bullet-shaped connecting structure, which is embedded into the accommodation slot 2122 provided at the tail of the latch 212 of the clip 210 to achieve mutual engagement.

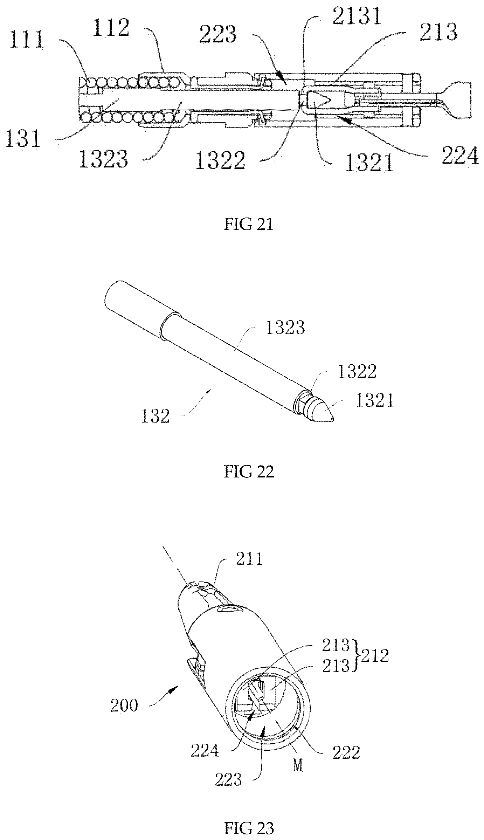

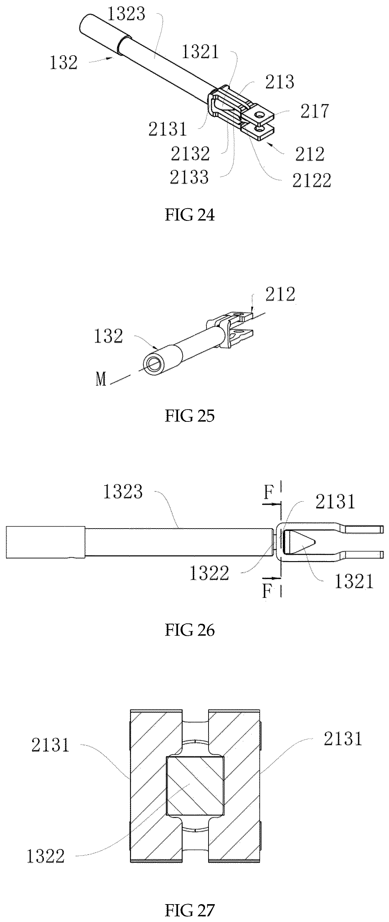

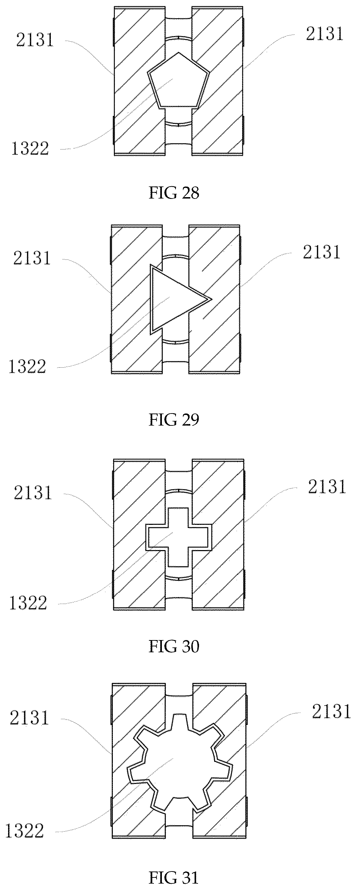

Optionally, referring to FIGS 21 and 22 together, in some embodiments, the transmission head 132 includes a cone head 1321, a joint part 1322, and a base 1323. The cone head 1321 has a roughly conical structure and can be inserted into the latch 212 of the clip 210 for connection with the clip 210. The base 1323 is connected to the cable 131 and spaced apart from the cone head 1321. The joint part 1322 is disposed between the base 1323 and the cone head 1321, and the base 1323 is connected to the cone head 1321 through the joint part 1322. The cross-section of the joint part 1322 perpendicular to the axial direction of the transmission assembly 130 is non-circular. In some embodiments, the cross-section of the joint part 1322 perpendicular to the axial direction of the transmission assembly 130 may also be circular. When the cone head 1321 is inserted into the clip 210, at least a portion of the clip 210 is located between the cone head 1321 and the base 1323, and is in contact with the joint part 1322. Since the joint part 1322 is non-circular, and at least a portion of the clip 210 is in contact with the joint part 1322, the transmission assembly 130 can transmit torque to the clip 210, thereby driving the clip 210 to rotate, improving the ease and flexibility of operation. Specifically, the non-circular shape can be polygonal, such as square, triangle, hexagon, etc., or it can be D-shaped, elliptical, star-shaped, multi-toothed, etc., as long as it can cooperate with the clip 210 to transmit torque. For example, as shown in FIG 27, the cross-section of the joint part 1322 is rectangular; as shown in FIG 28, the cross-section of the joint part 1322 is pentagonal; as shown in FIG 29, the cross-section of the joint part 1322 is triangular; as shown in FIG 30, the cross-section of the joint part 1322 is "+"-shaped; and as shown in FIG 31, the cross-section of the joint part 1322 is gear-shaped.

Referring to FIGS 6, 13, and 14 together, in some embodiments, the connecting assembly 120 includes an elastic part 121 and a tongue part 122. The elastic part 121 has a fixed end 1211 and a movable end 1212 disposed opposite to each other. The fixed end 1211 is fixed to the sheath assembly 110, and the tongue part 122 is connected to the movable end 1212 and extends from the movable end 1212 toward the lateral hole 114. The fixed end 1211 is the end of the elastic part 121 that is fixedly connected to the sheath assembly 110, and the movable end 1212 is the end that can move when the elastic part 121 is pressed by the transmission head 132 of the transmission assembly 130. When the elastic part 121 is not pressed, the movable end 1212 is closest to the central axis O. When the elastic part 121 is pressed, the movable end 1212 moves radially away from the central axis O, causing the tongue part 122 to protrude out of the lateral hole 114. When the elastic part 121 is not compressed, the tongue part 122 can be completely located within the channel 113, or partially located within the lateral hole 114, or slightly protruding outside the lateral hole 114. In this embodiment, when the transmission assembly 130 moves toward the inside of the clip seat 220, the transmission assembly 130 is capable of pressing the elastic part 121 to cause elastic deformation, so that the movable end 1212 moves radially away from the central axis O, causing the tongue part 122 to protrude from the lateral hole 114 to the outside of the sheath assembly 110 and connect with the clip seat 220. When the transmission assembly 130 moves toward the side where the proximal end 115 is located, its compressive force on the elastic part 121 gradually decreases, and the elastic deformation of the elastic part 121 gradually recovers, causing the movable end 1212 to gradually approach the central axis O, thereby causing the tongue part 122 to retract toward the lateral hole 114, thus separating from the clip seat 220. This way, the connection and separation of the delivery device 100 and the clip assembly 200 can be achieved simply by driving the transmission assembly 130, making it more convenient for doctors to operate.

Optionally, the elastic part 121 can be a spring piece structure with elasticity, which can be made of medical-grade metal or other medical-grade elastic materials. Furthermore, the elastic part 121 and the tongue part 122 can be an integral structure, that is, the two are integrally processed and formed.

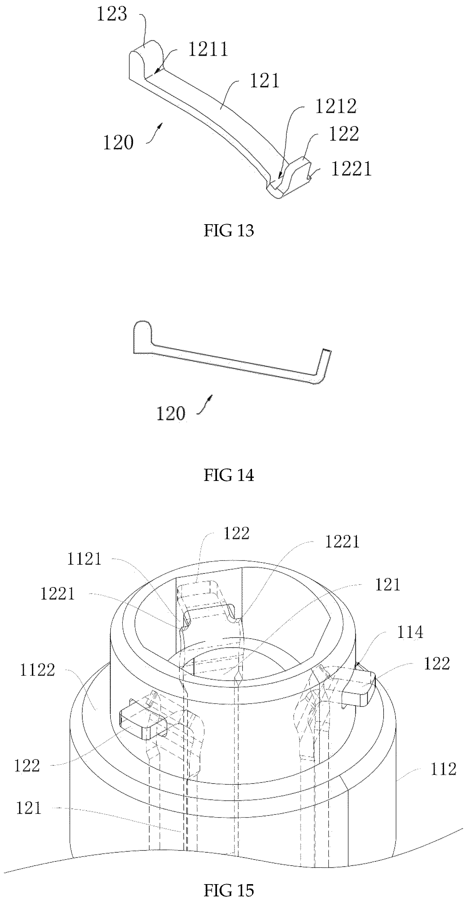

Optionally, as shown in FIGS 6 and 13, the elastic part 121 extends in a curved manner from the fixed end 1211 to the movable end 1212. Specifically, from the fixed end 1211 to the movable end 1212, the elastic part 121 extends curvedly with a tendency to approach roughly toward the central axis O of the sheath assembly 110.

Optionally, the distance between the fixed end 1211 and the central axis O is greater than the distance between the movable end 1212 and the central axis O.

Optionally, as shown in FIG 14, the elastic part 121 extends in a straight line from the fixed end 1211 to the movable end 1212.

Referring again to FIG 6, in some embodiments, the sheath assembly 110 also has a assembly hole 117 communicating with the channel 113. The assembly hole 117 may be formed on the transition cap 112. The connecting assembly 120 also includes a bending part 123, which is connected to the fixed end 1211. The bending part 123 is disposed within the assembly hole 117 and abuts against the hole wall 1171 of the assembly hole 117. By providing the bending part 123 at the fixed end 1211 and utilizing the bending part 123 to abut against the hole wall 1171 of the assembly hole 117, the assembly of the connecting assembly 120 and the sheath assembly 110 is simpler and the structure is more stable. The bending part 123 may be a U-shaped structure formed by bending, which, with its own elasticity, can be more stably held against the hole wall 1171 of the assembly hole 117, improving the stability of the assembly. Optionally, the elastic part 121, the tongue part 122, and the bending part 123 may be an integral structure, that is, the three are integrally processed and formed.

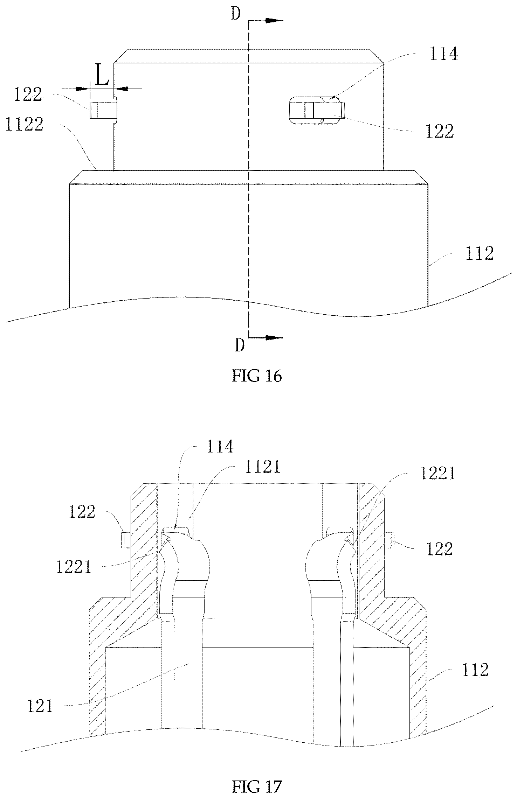

Referring to FIGS 13, 15, 16, and 17 together, in some embodiments, the tongue part 122 has a shoulder 1221 located inside the sheath assembly 110, i.e., within the channel 113. The shoulder 1221 is able to contact the side wall 1121 of the sheath assembly 110 to limit the position of the tongue part 122. It can be understood that, as shown in FIGS 16 and 17, the shoulder 1221 can limit the dimension L of the tongue part 122 protruding from the lateral hole 114 to the outside of the sheath assembly 110. Specifically, the shoulder 1221 is a protruding structure provided on the peripheral side of the tongue part 122, located within the channel 113. When the elastic part 121 is pressed and undergoes elastic deformation, the tongue part 122 protrudes outward from the lateral hole 114, and its shoulder 1221 can abut against the side wall 1121 of the sheath assembly 110, thereby limiting the protruding dimension L of the tongue part 122. Referring again to FIG 9, by limiting the protruding dimension of the tongue part 122 by the shoulder 1221, the depth of the tongue part 122 inserted into the limiting groove 222 can be limited, which can reduce friction with the clip seat 220 and improve the rotation freedom of the clip assembly 200.

Referring to FIG 13 again, shoulders 1221 are provided on two opposite sides of the tongue part 122. These shoulders 1221 is able to abut against the opposite sides of the lateral hole 114 respectively, improving the stability of the positioning. Optionally, the shoulders 1221 can also be configured as flange structures provided circumferentially around the tongue part 122.

In some embodiments, the sheath assembly 110 has a plurality of lateral holes 114 arranged circumferentially thereon, and the delivery device 100 includes a plurality of connecting assemblies 120, each connecting assembly 120 corresponding to one lateral hole 114. When the transmission assembly 130 moves toward the inside of the clip seat 220, the elastic part 121 of each connecting assembly 120 is pressed by the transmission assembly 130 and undergoes elastic deformation. By providing a plurality of lateral holes 114 circumferentially in the sheath assembly 110, the plurality of connecting assemblies 120 can be connected to the clip seat 220 by passing through the corresponding lateral holes 114 respectively, improving the stability of the connection between the delivery device 100 and the clip assembly 200. Furthermore, each connecting assembly 120 corresponds to one lateral hole 114, meaning that multiple connecting assemblies 120 are also arranged along the circumferential direction of the sheath assembly 110. This way, when the transmission assembly 130 moves, it can simultaneously apply compressive force to the elastic parts 121 of the multiple connecting assemblies 120 arranged circumferentially, making the interaction force between the elastic parts 121 and the transmission assembly 130 more balanced in the axial direction, maintaining the movement path of the transmission assembly 130, reducing its deviation, and thereby improving the accuracy of the connection between the transmission assembly 130 and the clip 210.

It is understood that the number of connecting assemblies 120 and lateral holes 114 is the same. Optionally, this number may be 1, 2, 3, 4 or even more, and can be specifically set as needed. Optionally, multiple connecting assemblies 120 and multiple lateral holes 114 may be evenly arranged along the circumference of the sheath assembly 110.

In conjunction with the above embodiments, a plurality of assembly holes 117 may be provided on the sheath assembly 110, the plurality of assembly holes 117 are arranged in the circumferential direction of the sheath assembly 110, and the bending part 123 of each connecting assembly 120 is installed in one assembly hole 117.

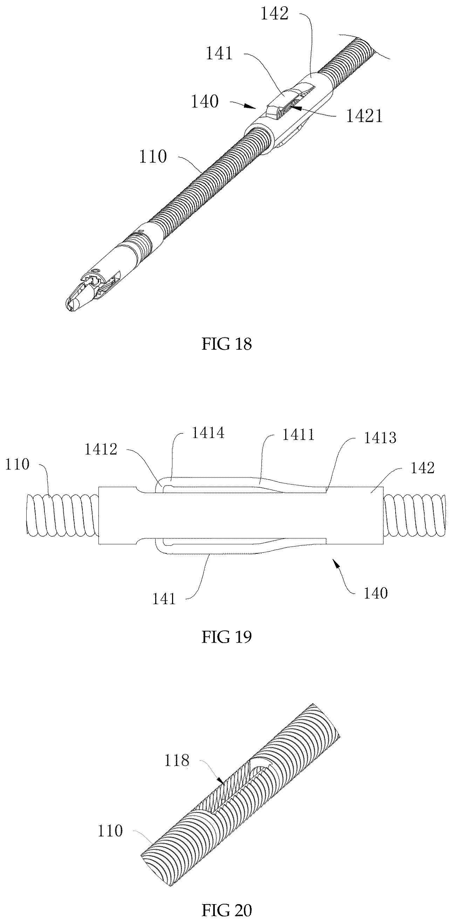

Referring to FIGS 18, 19, and 20, in some embodiments, the sheath assembly 110 further has avoidance grooves 118 communicating with the channel 113. The avoidance grooves 118 are located on opposite sides of the sheath assembly 110 in its radial direction and extend along the extending direction of the sheath assembly 110. Optionally, the avoidance grooves 118 are formed on opposite sides of the sheath body 111. In this embodiment, the delivery device 100 further includes an auxiliary assembly component 140, which is configured to move along the extending direction of the sheath assembly 110. The auxiliary assembly component 140 has a clamping part 141, which is disposed opposite to the avoidance grooves 118. It can be understood that the clamping part 141 includes two opposing parts, each corresponding to one avoidance groove 118. The clamping part 141 is configured to be able to enter the channel 113 through the avoidance grooves 118 when pressed, so as to clamp the transmission assembly 130.

It can be understood that, after the clip seat 220 is fixed with the transition cap 112 of the sheath assembly 110, a slider on the handle mechanism 150 needs to be pushed to move the transmission assembly 130 to the distal end 116, thereby pressing the connecting assembly 120 to connect the delivery device 100 and the clip seat 220 together. However, during the process of pushing the slider, because the sheath assembly 110 and the transmission assembly 130 are relatively long, the slider is far from the distal end 116, making operation inconvenient. By providing the auxiliary assembly component 140, the transmission assembly 130 can be clamped through the avoidance groove 118 by its clamping part 141, and the effect of moving the transmission assembly 130 can be achieved by moving the auxiliary assembly component 140, which greatly reduces the assembly difficulty and improves the assembly convenience.

Specifically, referring again to FIG 19, the auxiliary assembly component 140 includes a main body 142, which is sleeved on the sheath assembly 110 and can move along the extending direction of the sheath assembly 110. The clamping part 141 is connected to the main body 142. By providing the main body 142 sleeved on the sheath assembly 110, the sheath assembly 110 can be used to guide the main body 142, making the pushing direction of the auxiliary assembly component 140 on the transmission assembly 130 more accurate when the auxiliary assembly component 140 is moved, and making it easier to complete the assembly of the delivery device 100 and the clip assembly 200.

Optionally, as shown in FIG 18, the main body 142 has a relief groove 1421, which corresponds to the avoidance groove 118. At least a portion of the clamping part 141 is disposed within the relief groove 1421, and the clamping part 141 is configured such that at least a portion of it can pass through the relief groove 1421 and enter the avoidance groove 118 when pressed. By disposing at least a portion of the clamping part 141 within the relief groove 1421, a portion of the structure of the main body 142 is located at the front end of the clamping part 141, and another portion is located at the rear end of the clamping part 141, allowing the sheath assembly 110 to be sleeved on both the front and rear sides of the clamping part 141, improving its movement stability. Furthermore, during processing, the clamping part 141 can be directly stamped out on the main body 142 and the relief groove 1421 is formed.

In some embodiments, as shown in FIG 19, the clamping part 141 includes a drive arm 1411 and a pressing head 1412. The drive arm 1411 has a connecting end 1413 and a free end 1414 disposed opposite to each other. The connecting end 1413 is connected to the main body 142. From the connecting end 1413 to the free end 1414, at least a portion of the drive arm 1411 extends in a direction gradually away from the transmission assembly 130. The pressing head 1412 is connected to the free end 1414 and extends from the free end 1414 toward the transmission assembly 130. By pressing the drive arm 1411, the pressing head 1412 can be pressed onto the transmission assembly 130 to clamp the transmission assembly 130.

Accordingly, the clip device provided in the embodiment of the present disclosure includes the delivery device 100 and the clip assembly 200, wherein the delivery device 100 is configured to deliver the clip assembly 200. This clip device may possess all the technical features and effects of the delivery device 100 in the above embodiments, and will not be repeated here.

It should be noted that, in the clip device of the embodiment of the present disclosure, the clip assembly 200 and the delivery device 100 are configured as separate structures, and the two are connected together for use when needed.

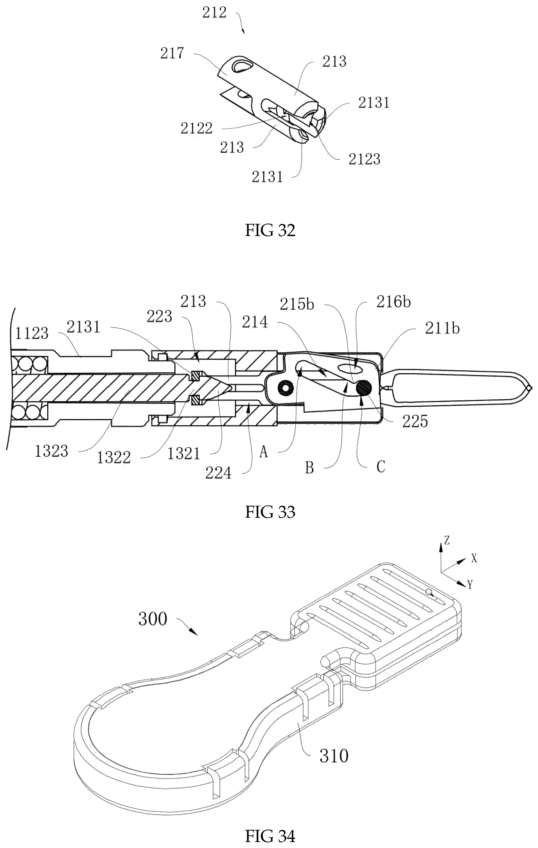

In order to assemble the clip assembly 200 and the delivery device 100, the clip device also includes a protective housing 300 as shown in FIG 34. The protective housing 300 is configured for assembling the clip assembly 200 and the delivery device 100. The protective housing 300 includes a positioning assembly 320. The clip assembly 200 can be housed within the protective housing 300, and the delivery device 100 can be inserted into the protective housing 300 and positioned in cooperation with the positioning assembly 320 so as to be able to connect with the clip assembly 200.

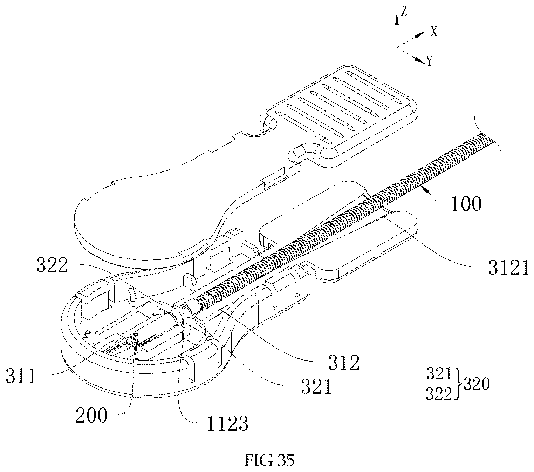

In some embodiments, referring to FIGS 34 and 35 together, the protective housing 300 further includes a housing assembly 310 internally defined a receiving slot 311 and an assembly slot 312, the receiving slot 311 being configured for placing the clip assembly 200, and the assembly slot 312 being configured for the delivery device 100 to be inserted into the receiving slot 311 therethrough. The positioning assembly 320 is connected to the housing assembly 310 and includes a first positioning part 321 and a second positioning part 322 disposed opposite to each other, the first positioning part 321 and the second positioning part 322 being disposed between the receiving slot 311 and the assembly slot 312. The first positioning part 321 and the second positioning part 322 are configured to cooperate with the sheath assembly 110 in the delivery device 100 to position the sheath assembly 110 when the delivery device 100 is inserted into the receiving slot 311.

In some embodiments, the groove wall of the assembly slot 312 near the opening of the housing assembly 310 is configured as a guide surface 3121. The guide surface 3121 is roughly conical and capable of accurately guiding the delivery device 100 so that the delivery device 100 can be accurately inserted into the interior of the protective housing 300.

In some embodiments, the transition cap 112 of the sheath assembly 110 is provided with a mating structure. The positioning of the sheath assembly 110 is achieved by the cooperation of the mating structure with the first positioning part 321 and the second positioning part 322. Optionally, the mating structure may be a limiting recess 1123 provided on the transition cap 112.

Referring to FIGS 2 and 3 together, the clip assembly 200 includes a clip 210 and a clip seat 220 that houses at least a portion of the clip 210. The clip 210 is configured to connect with the transmission assembly 130. The inner peripheral surface 221 of the clip seat 220 is provided with a limiting groove 222. The connecting assembly 120 can be embedded in the limiting groove 222 to connect with the clip seat 220. By providing a shoulder 1221 on the tongue part 122 of the connecting assembly 120, the depth of the tongue part 122 extending out of the lateral hole 114 can be controlled by the shoulder 1221. This reduces the contact area between the tongue part 122 and the groove wall of the limiting groove 222, lowers friction, and makes the clip assembly 200 rotate more flexibly.

Referring again to FIGS 2 and 3, in some embodiments, the clip 210 includes a clip arm element 211 and a latch 212 connected together. Referring to FIG 32, the latch 212 includes at least two retaining units 213 arranged around its central axis M. The end of the retaining unit 213 away from the clip arm element 211 has a retaining part 2131. As shown in FIG 9, the transmission head 132 of the transmission assembly 130 can be inserted between at least two retaining parts 2131 of the at least two retaining units 213. The retaining parts 2131 are configured to limit the position of the transmission head 132 so that the transmission head 132 is connected to the latch 212.

Optionally, in the embodiment shown in FIG 32, the latch 212 includes a hinge seat 217 and three retaining units 213 connected to one end of the hinge seat 217. The hinge seat 217 is hinged to the clip arm element 211, and each retaining unit 213 has a retaining part 2131. The three retaining units 213 are arranged at intervals around the central axis M, and a accommodation slot 2122 is formed between the three retaining units 213, into which the cone head 1321 of the transmission head 132 can be inserted. The retaining part 2131 is provided at the opening 2123 of the accommodation slot 2122, and all three retaining parts 2131 are provided to protrude inward along the radial direction of the latch 212. Referring to FIG 9, when the cone head 1321 is inserted into the accommodation slot 2122, the retaining part 2131 can be locked on the side of the cone head 1321 near the base 1323, limiting the position of the cone head 1321.

Optionally, in the embodiments shown in FIGS 21 to 27, the latch 212 includes a hinge base 217 and two retaining units 213 connected to one end of the hinge base 217. The hinge base 217 is hinged to the clip arm element 211. The two retaining units 213 are respectively disposed on two sides of the central axis M and spaced apart from each other. The retaining unit 213 also includes a first retaining arm 2132 and a second retaining arm 2133 respectively connected to the hinge base 217. A retaining part 2131 is disposed on the side of the first retaining arm 2132 and the second retaining arm 2133 away from the hinge base 217 and connects the first retaining arm 2132 and the second retaining arm 2133. The hinge base 217 includes two sheet-like structures, which are provided with pin holes and hinged with the clip arm element 211 through the pin holes. One sheet-like structure connects the first retaining arm 2132 of the two retaining units 213, and the other sheet-like structure connects the second retaining arm 2133 of the two retaining units 213.

As shown in FIGS 26 and 27, when the transmission head 132 is inserted between at least two retaining units 213, the retaining part 2131 is located between the cone head 1321 and the base 1323 of the transmission head 132, and is in contact with the joint part 1322. The cross-section of the joint part 1322 perpendicular to the axial direction of the transmission head 132 is non-circular. In some embodiments, the cross-section of the joint part 1322 perpendicular to the axial direction of the transmission assembly 130 may also be circular. This allows the transmission assembly 130 to transmit torque to the clip 210, thereby driving the clip assembly 200 to rotate. Specifically, the transmission head 132 drives the clip assembly 200 to rotate relative to the connecting assembly 120, improving the convenience and flexibility of operation. Combined with the shoulder 1221 controlling the depth of the tongue part 122 extending out of the lateral hole 114, the flexibility of rotation is further improved. Specifically, a gap is formed between the holding portions 2131 of the multiple retaining units 213 to accommodate the joint part 1322. The shape of this gap is generally non-circular, but it can also be circular and transmit torque through an interference fit. The specific shape can be selected according to the actual use. The non-circular shape can be polygonal, such as square, triangle, hexagon, etc., or it can be D-shaped, elliptical, star-shaped, multi-toothed, etc., as long as it can cooperate with the clip 210 to transmit torque. For example, as shown in FIG 27, the cross-section of the joint part 1322 is rectangular; as shown in FIG 28, the cross-section of the joint part 1322 is pentagonal; as shown in FIG 29, the cross-section of the joint part 1322 is triangular; as shown in FIG 30, the cross-section of the joint part 1322 is "+"-shaped; and as shown in FIG 31, the cross-section of the joint part 1322 is gear-shaped.

Referring again to FIG 3, the internal space of the clip seat 220 has a first section 223 and a second section 224 arranged sequentially along its axial direction. The first section 223 is located on the side of the second section 224 away from the clip arm element 211, and the radial dimension R of the first section 223 is greater than the radial dimension r of the second section 224. Here, "radial dimension" refers to the dimension of the inner contour of the structure along a direction perpendicular to the central axis of the clip assembly 200. This radial dimension is independent of the cross-sectional shape of the clip assembly 200 (such as circular, polygonal, or irregular shape), and is based solely on its central axis. For example, if the internal space is cylindrical, the radial dimension is the length in the diameter direction; and if the internal space is polygonal, the radial dimension is twice the vertical distance from the central axis to any side wall.

Referring to FIGS 9, 21, and 33, when the retaining part 2131 is located within the first section 223, the retaining parts 2131 of at least two retaining units 213 can be pushed away from each other by the pressing force of the transmission head 132. When the clip assembly 200 is fixed in the protective housing 300 in the closed state shown in FIGS 9 and 33, the retaining part 2131 is located within the first section 223. The movement of the transmission assembly 130 toward the distal end 116 of the sheath assembly 110 can press the retaining part 2131, causing the retaining units 213 to undergo elastic deformation, and the retaining parts 2131 move away from each other, so that the cone head 1321 of the transmission head 132 can be engaged between the retaining units 213, realizing the connection with the latch 212. When the clip arm element 211 is locked, and the retaining part 2131 is also located within the first section 223, the movement of the transmission assembly 130 toward the proximal end 115 of the sheath assembly 110 can press the retaining part 2131, causing the retaining unit 213 to undergo elastic deformation. The retaining parts 2131 move away from each other, so that the cone head 1321 of the transmission head 132 can disengage from the latch 212, releasing the clip assembly 200. Furthermore, the cone head 1321 is designed with a tapered structure. When the transmission head 132 is inserted into the latch 212, the tapered surface of the cone head 1321 contacts the latch 212 first, which can play a guiding role. Therefore, the force required to insert the latch 212 is very small, and it is relatively easy to insert the latch 212. When the transmission head 132 is pulled out of the latch 212, there is no tapered surface to guide it, so the pulling force is larger. This ensures that the clip arm element 211 enters the locked state.

In the embodiment shown in FIG 33, the transition cap 112 is also provided with a limiting recess 1123. When the delivery device 100 is inserted into the protective housing 300, it can be positioned by the cooperation of the limiting recess 1123 of the transition cap 112 with the structure inside the protective housing 300.

Referring to FIG 10, when the retaining part 2131 is located within the second section 224, the inner peripheral surface 221 of the clip seat 220 can contact the outer peripheral surface 2121 of the retaining unit 213 to prevent the retaining parts 2131 of at least two retaining units 213 from moving away from each other. In this state, the latch 212 is constrained by the inner peripheral surface 221 of the clip seat 220 and is difficult to generate sufficient deformation. Therefore, the cone head 1321 of the transmission head 132 is not easy to disengage from the latch 212, and it is difficult for the transmission head 132 to disengage from the latch 212 prematurely. This effectively solves the pain point problem in clinical practice-the situation where the clip arm element 211 fails to clip or clips off, improves the reliability of clamping, ensures that the clip arm element 211 can clamp larger wounds, and ensures the stability of surgical operations. When the clip assembly 200 is released, as shown in the state of FIG 9, the retaining part 2131 of the latch 212 is located in the first section 223. This part is not constrained by the inner peripheral surface 221, and it is easier to generate outward expansion deformation, that is, to move away from each other. In this state, the connection force between the transmission head 132 and the latch 212 is small, and it is easier to release.

The latch 212 is designed as an elastic structure, which allows it to undergo elastic deformation during the process of the cone head 1321 being engaged into the latch 212, thereby confining the cone head 1321 within the latch 212.

As shown in FIG 2, the clip arm element 211 of the clip assembly 200 includes a first clip arm 211a and a second clip arm 211b hinged to the latch 212. Both the first clip arm 211a and the second clip arm 211b are provided with a sliding groove 214. As shown in FIG 33, a pin 225 is provided in the clip seat 220, and the pin 225 passes through the sliding groove 214. The sliding groove 214 is provided with an open position A, a closed position B, and a locking position C. When the pin 225 is in the open position A, the first clip arm 211a and the second clip arm 211b are open, that is, the clip arm element 211 is in the open state. When the pin 225 is in the closed position B, the first clip arm 211a and the second clip arm 211b are closed, that is, the clip arm element 211 is in the closed state. When the pin 225 is in the locking position C, the first clip arm 211a and the second clip arm 211b are locked.

Specifically, the transmission assembly 130 drives the clip arm element 211 to move via the latch 212, so that the pin 225 is able to reciprocate between the open position A and the closed position B. The locking position C is located on the side of the closed position B away from the open position A. The first clip arm 211a has a first protrusion 215a in its sliding groove 214, and the second clip arm 211b has a second protrusion 215b opposite to the first protrusion 215a in its sliding groove 214. The first protrusion 215a and the second protrusion 215b are located between the locking position C and the closed position B. The transmission assembly 130 drives the clip arm element 211 to move toward the proximal end 115 via the latch 212, so that the pin 225 presses against the first protrusion 215a and the second protrusion 215b and moves to the locking position C.

In some embodiments, the first clip arm 211a is provided with a first avoidance notch 216a, and the second clip arm 211b is provided with a second avoidance notch 216b. The pin 225 presses against the first protrusion 215a and the second protrusion 215b, causing the first protrusion 215a to deform toward the first avoidance notch 216a and the second protrusion 215b to deform toward the second avoidance notch 216b. This makes it easier for the pin 225 to engage in the locking position C. When the pin 225 engages in the locking position C, the retaining part 2131 of the latch 212 moves to the first section 223. The force required for the pin 225 to pass through the first protrusion 215a and the second protrusion 215b and engage in the locking position C is less than the force required for the transmission head 132 to disengage from the latch 212.

The embodiment of the present disclosure also provides an assembly method for the clip device as described in any of the above embodiments. The assembly method includes:
inserting the delivery device 100 into the protective housing 300 so that the positioning assembly 320 of the protective housing 300 engages with the delivery device 100 to position the delivery device 100;
manipulating the handle mechanism 150 of the delivery device 100 to insert the transmission assembly 130 of the delivery device 100 into the clip seat 220 of the clip assembly 200 and connect it with the clip 210, and the clip assembly 200 is received in the protective housing 300 in a closed state; and
completing the assembly by pulling the delivery device 100 out of the protective housing 300, and taking the clip assembly 200 out together. The clip assembly 200 is accommodated within the protective housing 300 in a closed state to facilitate connection between the latch 212 and the transmission head 132. Specifically, during the insertion of the sheath assembly 110 and the transmission assembly 130 of the delivery device 100 into the protective housing 300, the transition cap 112 of the sheath assembly 110 presses against the first positioning part 321 and the second positioning part 322, causing them to elastically deform and move away from each other. When the distal end 116 of the transition cap 112 is inserted into the receiving slot 311, the first positioning part 321 and the second positioning part 322 clamp the transition cap 112 due to the elastic restoring force, thus positioning it. Optionally, a limiting recess 1123 is provided on the transition cap 112, and the first positioning part 321 and the second positioning part 322 engage with the limiting recess 1123 for positioning.

In the above embodiments, the description of each embodiment has its own emphasis. For parts not described in detail in a certain embodiment, reference can be made to the relevant descriptions of other embodiments.

The delivery device 100, clip device, and assembly method provided in the embodiments of the present disclosure have been described in detail above, and specific examples have been used to illustrate the principles and implementation methods of the present disclosure. The description of the above embodiments is only for the purpose of helping to understand the technical solutions and core ideas of the present disclosure. Those skilled in the art should understand that they can still modify the technical solutions described in the foregoing embodiments, or make equivalent substitutions for some of the technical features; and these modifications or substitutions do not make the essence of the corresponding technical solutions to deviate from the scope of the technical solutions of the embodiments of the present disclosure.

## Claims

1. A delivery device, configured to deliver a clip assembly comprising a clip and a clip seat for accommodating at least a portion of the clip, wherein the delivery device comprises:
a sheath assembly having a channel and a lateral hole, the channel extending through a proximal end and a distal end of the sheath assembly, and the lateral hole communicating with the channel;
a connecting assembly disposed within the channel and connected to the sheath assembly; and
a transmission assembly passing through the channel;
wherein the distal end of the sheath assembly is able to be inserted into the clip seat, and the transmission assembly is able to move along the channel toward the clip seat to connect with the clip; and
when the transmission assembly moves toward an inside of the clip seat, the transmission assembly is able to press the connecting assembly so that a portion of the connecting assembly protrudes from the lateral hole to an outside of the sheath assembly and connects with the clip seat.

2. The delivery device according to claim 1, wherein the transmission assembly is also capable of moving along the channel toward the proximal end of the sheath assembly to disengage from the clip; and
when the transmission assembly moves toward the proximal end of the sheath assembly, the connecting assembly is able to disengage from the clip seat.

3. The delivery device according to claim 1 or 2, wherein the connecting assembly comprises:
an elastic part having a fixed end and a movable end that are disposed opposite to each other, wherein the fixed end is fixed to the sheath assembly; and
a tongue part, which is connected to the movable end and extends from the movable end toward the lateral hole;
wherein when the transmission assembly moves toward the inside of the clip seat, the transmission assembly is capable of pressing the elastic part to cause it to elastically deform, thereby causing the tongue part to protrude from the lateral hole to the outside of the sheath assembly and connect with the clip seat.

4. The delivery device according to claim 3, wherein the sheath assembly has a central axis O; and
the distance between the fixed end and the central axis O is greater than the distance between the movable end and the central axis O.

5. The delivery device according to claim 3, wherein the tongue part has a shoulder located within the sheath assembly; and
the shoulder is able to contact the sidewall of the sheath assembly.

6. The delivery device according to claim 3, wherein the sheath assembly further has an assembly hole communicating with the channel; and
the connecting assembly further comprises a bending part connected to the fixed end, the bending part being disposed within the assembly hole and abutting against a hole wall of the assembly hole.

7. The delivery device according to claim 3, wherein the sheath assembly has a plurality of the lateral holes arranged circumferentially thereon; and
the delivery device comprises a plurality of the connecting assembly, and the tongue part of each connecting assembly is provided corresponding to one of the lateral holes.

8. The delivery device according to claim 1 or 2, wherein the sheath assembly further has an avoidance groove communicating with the channel, the avoidance groove being located on opposite sides of the sheath assembly in its radial direction and extending along the extension direction of the sheath assembly; and the delivery device further comprises:
an auxiliary assembly component, configured to move along the extension direction of the sheath assembly, wherein the auxiliary assembly component is provided with a clamping part disposed opposite to the avoidance groove, and the clamping part is configured to be able to enter the channel through the avoidance groove when pressed, so as to clamp the transmission assembly.

9. The delivery device according to claim 8, wherein the auxiliary assembly component includes a main body that is sleeved on the sheath assembly and is movable along the extending direction of the sheath assembly, and the clamping part is connected to the main body;
the main body has a relief groove, which is provided corresponding to the avoidance groove; and
at least a portion of the clamping part is disposed within the relief groove, and the clamping part is configured such that when pressed, at least a portion thereof is able to pass through the relief groove into the avoidance groove.

10. The delivery device according to claim 9, wherein the clamping part comprises:
a drive arm having a connecting end and a free end disposed opposite to each other, the connecting end being connected to the main body; and from the connecting end to the free end, at least a portion of the drive arm extends in a direction gradually away from the transmission assembly; and
a pressing head, which is connected to the free end and extends from the free end toward the transmission assembly.

11. The delivery device according to claim 1 or 2, wherein the sheath assembly comprises a sheath body and a transition cap arranged sequentially from its proximal end to its distal end and connected to each other, the transmission assembly passing through the sheath body and the transition cap, the lateral hole being formed on the transition cap, and at least a portion of the connecting assembly being located within the transition cap.

12. The delivery device according to claim 11, wherein the end of the transition cap away from the sheath body is the distal end of the sheath assembly, the transition cap is provided with a limiting step, and the lateral hole is located on the side of the limiting step closer to the distal end; and
when the distal end of the sheath assembly is inserted into the clip seat, the lateral hole is located within the clip seat, and the limiting step is opposite to an edge of the clip seat.

13. The delivery device according to claim 11, wherein the transmission assembly comprises a cable and a transmission head connected together, the cable being configured to drive the transmission head to move along the channel, and the transmission head being configured to press the connecting assembly and to connect with the clip.

14. The delivery device according to claim 13, wherein the transmission head comprises:
a cone head configured to be inserted into the clip;
a base, which is connected to the cable and spaced apart from the cone head; and
a joint part connected between the base and the cone head;
wherein when the cone head is inserted into the clip, at least a portion of the clip is located between the cone head and the base and is in contact with the joint part.

15. A clip device, comprising:
the delivery device according to any one of claims 1 to 14;
a clip assembly, wherein the clip assembly and the delivery device are configured as separate structures; and
a protective housing for assembling the clip assembly and the delivery device, wherein the protective housing comprises a positioning assembly;
wherein the clip assembly is able to be received within the protective housing, and the delivery device is able to be inserted into the protective housing and positioned in conjunction with the positioning assembly to connect with the clip assembly.

16. The clip device according to claim 15, wherein the clip assembly comprises a clip and a clip seat for receiving at least a portion of the clip, the clip being configured to connect to a transmission assembly of the delivery device, an inner peripheral surface of the clip seat being provided with a limiting groove, and a connecting assembly of the delivery device being able to be embedded in the limiting groove to connect to the clip seat.

17. The clip device according to claim 16, wherein the clip comprises a clip arm element and a latch connected together, the latch comprising at least two retaining units arranged about its central axis M, the retaining unit having a retaining part at an end away from the clip arm element; and
the transmission head of the transmission assembly is able to be inserted between the at least two retaining units, and the retaining part is configured to limit a position the transmission head so that the transmission head is connected to the latch.

18. The clip device according to claim 17, wherein an internal space of the clip seat has a first section and a second section arranged sequentially along its axial direction, the first section being located on a side of the second section away from the clip arm element, and a radial dimension R of the first section being greater than the radial dimension r of the second section;
when the retaining part is located within the first section, the retaining parts of the at least two retaining units is able to be moved away from each other by a pressing force of the transmission head; and
when the retaining part is located within the second section, the inner peripheral surface of the clip seat is able to contact the retaining unit to prevent the retaining parts of the at least two retaining units from moving away from each other.

19. The clip device according to claim 17, wherein a receiving slot is formed between the at least two retaining units, the transmission head is able to be inserted into the receiving slot, and the retaining part is disposed at an opening of the receiving slot and protrudes radially inward.

20. The clip device according to claim 17, wherein the latch is configured as an elastic structure and further comprises a hinge seat hinged to the clip arm element, and the retaining unit is disposed on the side of the hinge seat away from the clip arm element; and
the retaining unit further comprises a first retaining arm and a second retaining arm respectively connected to the hinge seat, and the retaining part is disposed on the side of the first retaining arm and the second retaining arm away from the hinge seat and connects the first retaining arm and the second retaining arm.

21. The clip device according to claim 17, wherein, when the transmission head is inserted between the at least two retaining units, the retaining part is located between the cone head and the base of the transmission head and is in contact with the joint part.

22. The clip device according to claim 21, wherein the transmission head is capable of driving the clip assembly to rotate relative to the connecting assembly.

23. The clip device according to claim 17, wherein the clip arm element comprises a first clip arm and a second clip arm hinged to the latch, both the first clip arm and the second clip arm are provided with a sliding groove, and a pin is provided in the clip seat and passes through the sliding groove;
the sliding groove is provided with an open position A, a closed position B and a locking position C, and the transmission assembly drives the clip arm element to move through the latch so that the pin is able to reciprocate between the open position A and the closed position B, wherein in the open position A, the first clip arm and the second clip arm are open, and in the closed position B, the first clip arm and the second clip arm are closed; and
the locking position C is located on the side of the closed position B away from the open position A, the first clip arm is provided with a first protrusion in its sliding groove, and the second clip arm is provided with a second protrusion in its sliding groove opposite to the first protrusion, and the first protrusion and the second protrusion are located between the locking position C and the closed position B, wherein the transmission assembly drives the clip arm element to move toward the proximal end through the latch, so that the pin presses the first protrusion and the second protrusion and moves to the locking position C, where the first clip arm and the second clip arm are locked.

24. The clip device according to claim 23, wherein the first clip arm is provided with a first avoidance notch, and the second clip arm is provided with a second avoidance notch, and the pin presses the first protrusion and the second protrusion so that the first protrusion deforms toward the first avoidance notch and the second protrusion deforms toward the second avoidance notch.

25. The clip device according to claim 15, wherein the protective housing further comprises:
a housing assembly, internally defining a receiving slot and an assembly slot, wherein the receiving slot is configured for placing the clip assembly, and the assembly slot is configured to allow the delivery device to be inserted into the receiving slot therethrough;
wherein the positioning assembly is connected to the housing assembly and comprises a first positioning part and a second positioning part arranged opposite to each other, and the first positioning part and the second positioning part are disposed between the receiving slot and the assembly slot, and are configured to engage with the sheath assembly in the delivery device to position the sheath assembly when the delivery device is inserted into the receiving slot.

26. An assembly method applied to the clip device according to any one of claims 15 to 25, wherein the assembly method comprises:
inserting the delivery device into the protective housing so that the positioning assembly of the protective housing cooperates with the delivery device to position the delivery device;
manipulating a handle mechanism of the delivery device to insert the transmission assembly of the delivery device into the clip seat of the clip assembly and connect it with the clip, and the clip assembly is received in the protective housing in a closed state; and
completing the assembly by pulling the delivery device out of the protective housing and taking the clip assembly out together.
